# EUROPEAN PATENT SPECIFICATION

(11) **EP 4 166 163 B1**
(45) Date of publication and mention of the grant of the patent: **01.07.2026**
(21) Application number: 21843165.8
(22) Date of filing: 16.07.2021
(51) Int. Cl.: A61L 2/10, A61L 9/20, F21V 15/01, A61L 2/084

(54) **STERILIZATION DEVICE**
STERILISATIONSVORRICHTUNG
DISPOSITIF DE STÉRILISATION

(30) Priority: 16.07.2020 KR 20200088156
(43) Date of publication of application: 19.04.2023
(73) Proprietor: Seoul Semiconductor Co., Ltd., Gyeonggi-do 15429 (KR)
(72) Inventor: LEE, Jang Weon, Ansan-Si Gyeonggi-do 15429 (KR)
(74) Representative: Stolmár & Partner Patentanwälte PartG mbB
(86) International application number: PCT/KR2021/009171
(87) International publication number: WO 2022/015100

(56) References cited:
- EP-A1- 3 132 189
- EP-A2- 1 069 441
- CN-A- 110 242 899
- JP-A- 2010 119 777
- KR-A- 20120 055 640
- KR-A- 20150 024 718
- KR-A- 20200 057 399
- KR-B1- 101 939 355
- US-A1- 2019 086 057

## Description

### [Technical Field]

Embodiments of the present disclosure relate to a sterilization device and, more particularly, to a sterilization device suitable for sterilizing bacteria or viruses in a room.

### [Background Art]

Pathogenic microorganisms, such as viruses and bacteria, cause various diseases in humans and animals. Furthermore, pathogenic microorganisms are transmitted between humans or between animals and humans by various routes. In particular, pathogenic microorganisms multiply within an infected person and infect another person through respiratory droplets produced by the infected person or physical contact with the infected person.

In order to prevent infection by pathogenic microorganisms, it is necessary to isolate infected individuals and to sterilize pathogenic microorganisms released from infected individuals.

Sterilization refers to any process that inactivates or kills pathogenic microorganisms, and targets a source of pathogenic microorganisms, such as various surfaces and objects where pathogenic microorganisms live. Such surfaces or objects generally include surfaces or objects that are touched by infected individuals or come into contact with respiratory droplets from infected individuals.

In general, sterilization uses chemical agents that can inactivate microorganisms. A typical disinfection operation is carried out by spraying such chemical agents into an area where pathogenic microorganisms are spread. However, use of these chemical agents is limited since the chemical agents are generally liquid and can leave residues containing a substance harmful to humans. Moreover, it is becoming increasingly difficult to treat bacteria with chemical agents due to emergence of many different antibiotic resistant bacteria in nature.

Sterilization using light, such as UV light, is also well known in the art. In particular, UV light having a wavelength of about 200 nm to about 320 nm is known to be very effective in inactivating or killing microorganisms. Surgical instruments, packaging containers, food containers, and the like are sterilized using a specially designed UV sterilizer. Such a UV sterilizer is adapted to sterilize pathogenic microorganisms on the surface of various objects in a small space using UV light.

Recently, an area sterilizer using a UV lamp is used in hospital rooms, hotel guest rooms, and the like. Such an area sterilizer generally uses a gas discharge lamp such as a mercury vapor lamp. The gas discharge lamp includes a tube containing a gas, such as mercury vapor, and a high-voltage applying device that generates an electrical discharge in the gas contained in the tube. The mercury vapor emits light having an intensity peak at a wavelength of about 253.7 nm by the electrical discharge to sterilize microorganisms.

The gas discharge lamp is used to disinfect an indoor space, such as a hospital room or a guest room, which is relatively large in area. However, the gas discharge lamp is expensive and requires a large device size for generating an electrical discharge in the gas. Moreover, the gas discharge lamp can have harmful effects on the environment due to use of mercury vapor. Furthermore, in order to disinfect many hospital rooms or guest rooms one after another, a support structure is needed to move the area sterilizer from one room to another room. However, this support structure makes it difficult to sterilize an area directly below the gas discharge lamp.

On the other hand, a light emitting diode emitting UV light suitable for sterilization has been developed. Such an inorganic semiconductor light source may also be used in UV sterilization devices.

The disclosure of US 2019/086057 A1 comprises a lighting apparatus including a heat radiation body comprising reflection sheets which distribute and redirect light emitted from an LED through a transparent window.

Document EP 1 069 441 A2 comprises the disclosure of a radiation system comprising reflectors encompassing flash lamps. The reflectors are configured to redirect light emitted from the lamps towards a target.

Publication CN 110 242 899 discloses a blackboard lamp lighting system. A reflective lens used within the system has a concave surface and reflects light emitted from an LED towards a blackboard retrograde to the LED light emission direction.

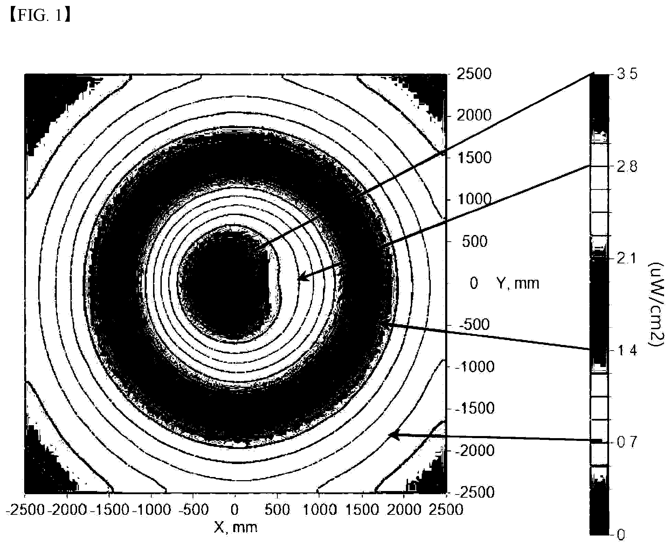

FIG. 1 is a simulation graph of an illuminance distribution produced by a typical UV light emitting diode. Specifically, illuminance levels on a floor were simulated under illumination by four light emitting diodes disposed at a height of 2 m from the center of the 5 m×5 m floor, followed by plotting a contour map by connecting points having the same illuminance.

Referring to FIG. 1, the light emitting diodes illuminate the floor in a concentric circular pattern. Accordingly, the illuminance level is highest at a central region of the floor and is lowest at corner regions of the floor.

In the simulation graph of FIG. 1, the central region of the floor has an illuminance of about 3.5 µW/cm² and the corner regions of the floor have an illuminance of 0.35 µW/cm². That is, the illuminance at the central region is about 10 times as high as that at the corner regions. Accordingly, when an optical output of the light emitting diode is adjusted to produce an illuminance suitable for sterilizing the corner regions, the central region will be illuminated at an unnecessarily high intensity. Conversely, when the optical output of the light emitting diode is adjusted to produce an illuminance suitable for sterilizing the central region, the illuminance at the corner regions will be insufficient for sterilization.

In conclusion, since an indoor space generally has a rectangular shape, a typical light emitting diode lighting device that produces a circular illumination pattern as shown in FIG. 1 has difficulty in sterilizing corners of the indoor space. Moreover, since UV illumination using a light emitting diode causes an excessively large illuminance difference between a central region and a peripheral region in an indoor space, significant energy loss is inevitable to achieve sufficient sterilization.

### [Disclosure]

### [Technical Problem]

Embodiments of the present disclosure provide an LED sterilization device suitable for sterilizing an indoor space.

Embodiments of the present disclosure provide a sterilization device capable of uniformly illuminating a large area with UV light.

Embodiments of the present disclosure provide a sterilization device which has a simple structure while ensuring effective sterilization of an indoor space.

### [Technical Solution]

The present invention is defined in the appended claims. In accordance with one aspect of the present disclosure, a sterilization device includes: a light emitting module; and a reflector reflecting light emitted from the light emitting module, in which the light emitting module includes a light emitting diode emitting sterilizing light, the reflector includes a concave reflective surface reflecting the light emitted from the light emitting module towards a horizontal surface below the light emitting module, and the concave reflective surface has a smaller radius of curvature near one end of a major diameter thereof than near the other end of the major diameter thereof.

Herein, a major axis direction X refers to a direction of a maximum diameter Dx of the reflector and a minor axis direction Y refers to a direction of a maximum diameter Dy of the reflector in an orthogonal direction to the major axis direction X. The major axis direction X and the minor axis direction Y pass through apexes of the reflector in two orthogonal directions, respectively. Here, the diameter Dx is referred to as a major diameter and the diameter Dy is referred to as a minor diameter.

Herein, the sterilizing light includes UV light or visible light having any wavelength that can kill or inactivate bacteria or viruses, including UV light in the wavelength range of 200 nm to 320 nm. For example, the sterilizing light may include UV light in the wavelength range of 320 nm to 390 nm or short wavelength visible light in the wavelength range of 390 nm to 430 nm.

According to the invention, the concave reflective surface is disposed above the light emitting module such that the major diameter thereof extends over the light emitting module; and the light emitting module is disposed closer to the one end of the major diameter of the concave reflective surface than to the other end of the major diameter of the concave reflective surface.

Further, a major axis direction of the concave reflective surface is tilted with respect to the horizontal surface such that the one end of the major diameter of the concave reflective surface is closer to the horizontal surface than the opposite end of the major diameter of the concave reflective surface.

Also, an uppermost point of the concave reflective surface tilted with respect to the horizontal surface is located between a center of the major diameter of the concave reflective surface and a reference point of the concave reflective surface located directly above the light emitting module.

The concave reflective surface may have a minor diameter orthogonal to the major diameter, the minor diameter being parallel to the horizontal surface.

The concave reflective surface may include an inner reflective surface reflecting the light emitted from the light emitting module towards a rectangular area on the horizontal surface.

The rectangular area may be square.

A light beam reflected from the reference point of the concave reflective surface located directly above the light emitting module may be directed to a center of the rectangular area.

A light beam reflected from the uppermost point of the concave reflective surface may be delivered to a region between the center of the rectangular area and a vertical projection point of the light emitting module on the rectangular area.

A light beam reflected from an edge of the inner reflective surface closest to the one end of the major diameter of the concave reflective surface among edges of the inner reflective surface may be delivered to a vertex of the rectangular area farthest from the light emitting module among vertices of the rectangular area; and a light beam reflected from an edge of the inner reflective surface closest to the other end of the major diameter of the concave reflective surface among the edges of the internal reflection surface may be delivered to a vertex of the rectangular area closest to the light emitting module among the vertices of the rectangular area.

The sterilization device may further include: a support supporting the light emitting module. The support may be a heat sink.

A maximum illuminance on the horizontal surface illuminated with the sterilizing light may be less than twice as high as an illuminance at a point on the horizontal surface illuminated with a light beam reflected from a reference point of the concave reflective surface located directly above the light emitting module.

The sterilization device may include: four light emitting modules; and four reflectors each reflecting light emitted from a corresponding one of the four light emitting modules.

The four light emitting modules and the four reflectors may be arranged to illuminate four quadrants of the horizontal surface about the sterilization device, respectively.

An illuminance at a center of the horizontal surface illuminated with the sterilizing light may be less than twice as high as an illuminance at a point on the horizontal surface illuminated with a light beam reflected from a reference point of the concave reflective surface located directly above each of the light emitting modules.

In accordance with another aspect of the present disclosure, a sterilization device includes: four light emitting modules; and four reflectors each reflecting light emitted from a corresponding one of the four light emitting modules, in which each of the light emitting modules includes a light emitting diode emitting sterilizing light, each of the reflectors includes a concave reflective surface reflecting light emitted from a corresponding one of the light emitting modules toward a horizontal surface below the light emitting modules, and the four light emitting modules and the four reflectors illuminate the horizontal surface about a region of the horizontal surface directly below the light emitting modules.

The concave reflective surface of each of the reflectors may have a major diameter and a minor diameter, the major diameter being tilted with respect to the horizontal surface, the minor diameter being parallel to the horizontal surface.

The sterilization device may further include: a heat sink, in which the four light emitting modules may be equidistantly arranged in a radial pattern on the heat sink.

The concave reflective surface of each of the reflectors may include an inner reflective surface reflecting the light towards a rectangular area on the horizontal surface.

The concave reflective surface of each of the reflectors may include a reference point located directly above a corresponding one of the light emitting modules; and an illuminance at a center of the horizontal surface may be less than twice as high as an illuminance at a reference point on the horizontal surface illuminated with a light beam reflected from the reference point of the concave reflective surface.

### [Advantageous Effects]

The sterilization device according to the embodiments is suitable for sterilizing an indoor space by reflecting light emitted from the light emitting diode towards a rectangular illumination target area using the reflector. In addition, due to a simple structure thereof, the sterilization device according to the embodiments can sterilize a large indoor space without requiring a large footprint.

The above and other advantages and features of the present disclosure will become apparent to those skilled in the art from the detailed description of the following embodiments.

### [Description of Drawings]

FIG. 1 is a simulation graph of an illuminance distribution produced by a typical UV light emitting diode.
FIG. 2 is a front view of a sterilization device according to one embodiment of the present disclosure.
FIG. 3 is a partially enlarged perspective view of the sterilization device according to the embodiment.
FIG. 4 is a plan view of the sterilization device according to the embodiment.
FIG. 5 is a schematic sectional view taken along line A-A of FIG. 4.
FIG. 6 is a view illustrating a beam propagation path of the sterilization device according to the embodiment.
FIG. 7 is a partially enlarged sectional view illustrating a beam propagation path of the sterilization device according to the embodiment.
FIG. 8 is a perspective view illustrating an illumination area produced by a combination of one light emitting diode and one reflector according to one embodiment of the present disclosure.
FIG. 9 is a schematic plan view illustrating a reflector according to one embodiment of the present disclosure.
FIG. 10 is a plan view illustrating an illumination area produced by the reflector according to the embodiment.
FIG. 11 is a schematic perspective view illustrating an illumination area produced by a UV sterilization device according to one embodiment of the present disclosure.
FIG. 12A, FIG. 12B, FIG. 12C, and FIG. 12D are respective simulation graphs of illuminance distributions on a floor produced by one, two, three, and four reflectors.
FIG. 13 is another simulation graph of an illuminance distribution on the floor produced by four reflectors.
FIG. 14 is a schematic sectional view illustrating an exemplary application of the sterilization device according to the embodiment.
FIG. 15 is a schematic sectional view illustrating another exemplary application of the sterilization device according to the embodiment.
FIG. 16 is a schematic perspective view of a sterilization device according to another embodiment of the present disclosure.

### [Best Mode]

Hereinafter, embodiments of the present disclosure will be described with reference to the accompanying drawings. It should be understood that the embodiments are provided for complete disclosure and thorough understanding of the present disclosure by those skilled in the art. Therefore, the present disclosure is not limited to the following embodiments and may be embodied in different ways. In addition, the drawings may be exaggerated in width, length, and thickness of components for descriptive convenience and clarity only. When an element is referred to as being "on," or "connected to" another element, it may be directly on or connected to the other element, or an intervening element may be present. Like components will be denoted by like reference numerals throughout the specification.

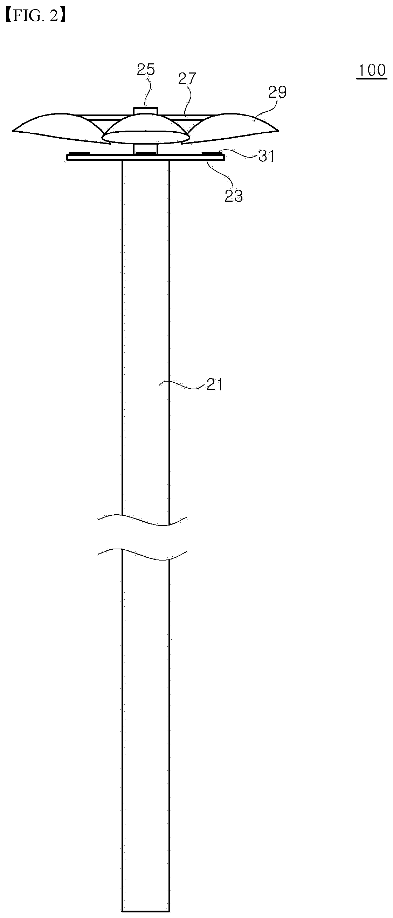

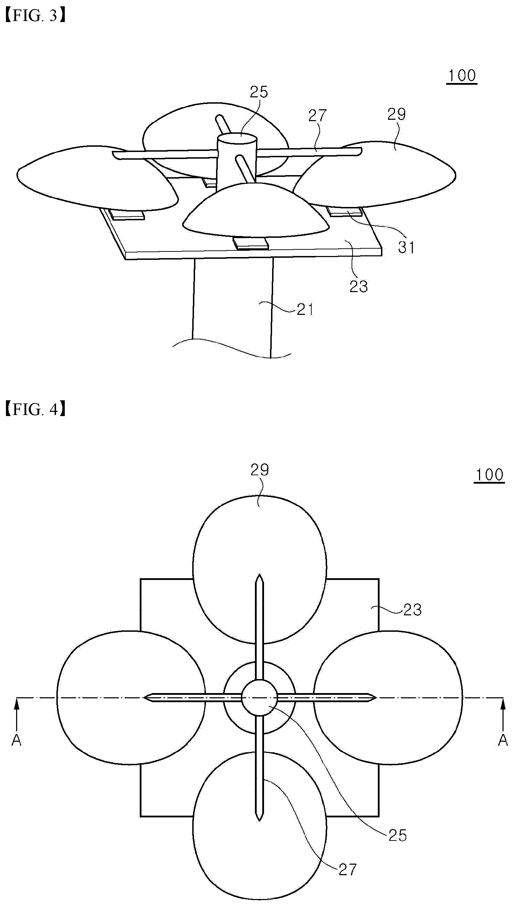

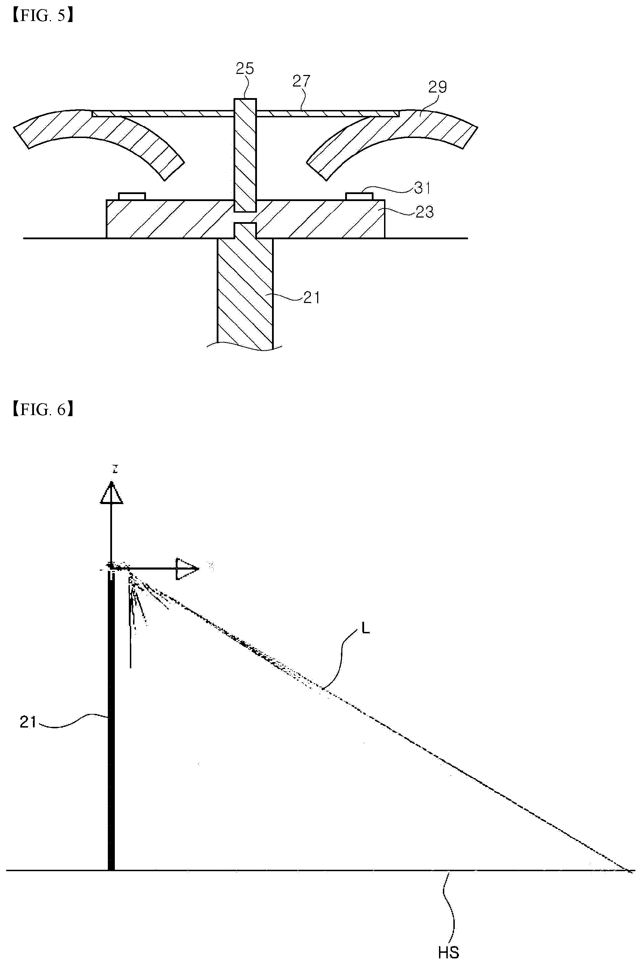

FIG. 2 is a front view of a sterilization device 100 according to one embodiment of the present disclosure, FIG. 3 is a partially enlarged perspective view of the sterilization device 100 according to the embodiment, FIG. 4 is a plan view of the sterilization device 100 according to the embodiment, and FIG. 5 is a schematic sectional view taken along line A-A of FIG. 4.

Referring to FIG. 2 to FIG. 5, the sterilization device 100 includes multiple light emitting modules 31 and multiple reflectors 29. In addition, the sterilization device 100 may include a lower column 21, a support 23, an upper column 25, and a connecting beam 27.

The lower column 21 is provided to install the sterilization device 100 in a specific space in a room, and may have various shapes such as a circular cylinder, a rectangular cylinder, or a hexagonal cylinder. The lower column 21 may have an elongated shape.

The lower column 21 may be coupled to a supporting structure such as a tripod or to a fixture in a room to be erected in an indoor space. The sterilization device 100 can be easily moved from one indoor space to another indoor space using the lower column 21.

In this embodiment, the lower column 21 supports the light emitting modules 31 while minimizing blockage of light caused thereby. Apart from the lower column 21, any other structure that is suitable for installing the sterilization device in an indoor space may be employed. For example, a support that can be attached to the ceiling, such as a pendant, may be used instead of the lower column 2 to hold the light emitting modules 31 and the reflectors 29 in an indoor space.

The support 23 supports the light emitting modules 31. The support 23 may have a plate-like shape, as shown in the drawings. In addition, the support 23 may also serve as a heat sink that dissipates heat generated by the light emitting modules 31. For example, the support 23 may be formed of a metal, for example, aluminum. Although the support 23 may have a generally rectangular shape, for example, a square shape, it should be understood that the present disclosure is not limited thereto and the support 23 may have various other shapes.

The support 23 may have various features that can improve heat dissipation. For example, the support 23 may include heat dissipation fins. Alternatively, the support 23 may include heat dissipation holes.

The upper column 25 may be coupled to an upper surface of the support 23 to be erected from the support 23 in an upward direction. The upper column 25 may have various shapes such as a circular cylinder, a rectangular cylinder, and a hexagonal cylinder. Instead of the cylindrical upper column 25, a supporting structure having a different shape may be coupled to the support 23.

The connecting beam 27 is secured to the upper column 25 to support the reflector 29. For example, four connecting beams 27 may be secured to the upper column 25 to support respective reflectors 29. The connecting beam 27 may have any shape that is suitable for supporting the reflector 29.

The light emitting modules 31 are arranged on the support 23. The light emitting modules 31 may be equidistantly arranged in a radial pattern with respect to the lower column 21 or the upper column 25. Each of the light emitting modules 31 may include a light emitting diode. The light emitting diode may emit light suitable for sterilizing viruses, bacteria, or the like. For example, the light emitting diode may emit UV light having a peak in the wavelength range of 200 nm to 320 nm, particularly UV light having a peak at a wavelength of about 275 nm. Alternatively, the light emitting diode may emit UV light having a peak in the wavelength range of 320 nm to 390 nm or visible light having a peak in the wavelength range of 390 nm to 430 nm.

Unlike mercury vapor discharge lamps, the light-emitting diode can emit short-wavelength light having a narrow full width at half maximum without emission of light unnecessary for sterilization, thereby reducing energy loss.

The reflector 29 reflects light emitted from the light emitting module 31 towards a horizontal surface below the light emitting module 31. The reflector 29 and the light emitting module 31 may be disposed to target a horizontal surface vertically spaced a predetermined distance apart from the light emitting module 31. For example, a distance from the light emitting module 31 to the targeted horizontal surface may be greater than the height of the average person, and may be, for example, about 2 m.

The reflector 29 may be disposed above the light emitting module 31 to cover the light emitting module 31. Referring to FIG. 5, the reflector 29 may be disposed such that a portion thereof is located directly above the support 23 and the other portion thereof is located outside a region directly above the support 23. In some embodiments, one end of the reflector 29 may be closer to the light emitting module 31 than the opposite end of the reflector 29.

The reflector 29 has a concave reflective surface that reflects light from the light emitting module 31. The concave reflective surface is a freeform reflector, and may include reflective surfaces of various curvatures. The various curvatures of the concave reflective surface are designed to reflect light emitted from the light emitting module 31 towards a specific region on the horizontal surface. Since an upper surface of the reflector 29 does not affect an optical path of light from the light emitting module 31, the shape thereof does not need to be specified. However, in order to reduce material costs, the reflector 29 may be formed to have a constant thickness. Thus, the upper surface of the reflector 29 may have a generally similar shape to the concave reflective surface. The shape and function of the concave reflective surface of the reflector 29 will be described in detail further below with reference to FIG. 6 to FIG. 10.

Each of the light emitting modules 31 pairs with a corresponding one of the reflectors 29. Each light emitting module-reflector pair may be disposed to illuminate a corresponding quarter of the horizontal surface. Accordingly, four light emitting module 31-reflector 29 pairs can illuminate four quadrants about the lower column 21. This type of illumination is suitable for sterilizing a generally rectangular space, such as a hospital room, a hotel guest room, and a bedroom or living room of a house.

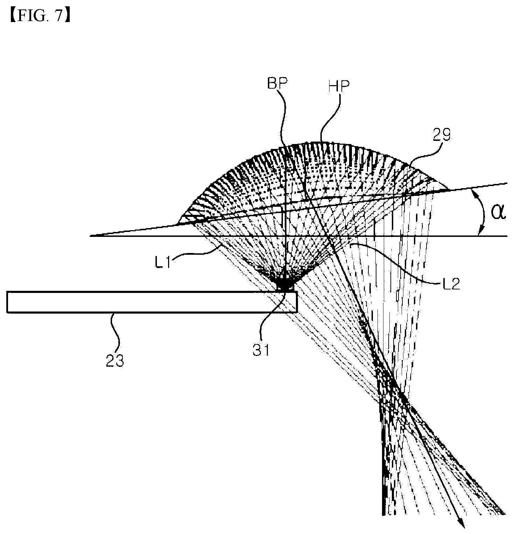

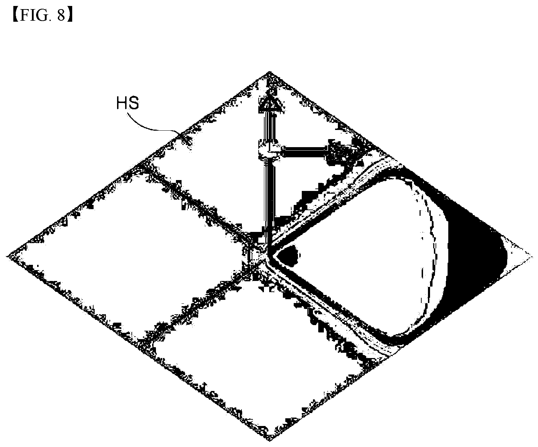

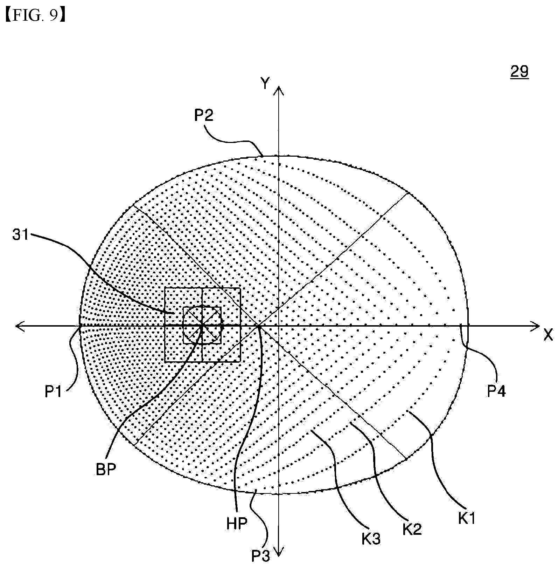

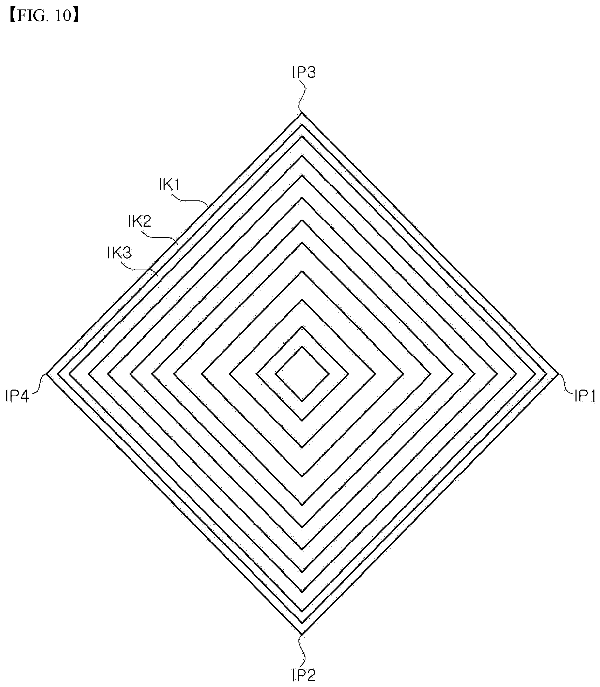

FIG. 6 is a sectional view illustrating a beam propagation path of the sterilization device according to the embodiment, FIG 7 is a partially enlarged sectional view illustrating a beam propagation path of the sterilization device according to the embodiment, FIG. 8 is a perspective view illustrating an illumination area produced by a combination of one light emitting diode and one reflector pair according to one embodiment of the present disclosure, FIG. 9 is a schematic plan view illustrating a reflector according to one embodiment of the present disclosure, and FIG. 10 is a plan view illustrating an illumination area produced by the reflector according to the embodiment.

Referring to FIG. 6 and FIG. 7, a horizontal surface HS is illuminated with light L reflected from the reflector 29 after being emitted from the light emitting module 31 disposed on the support 23. One of quadrants with respect to the lower column 21 is illuminated with the light L, as schematically shown in FIG. 8. Accordingly, all four quadrants can be illuminated using four light emitting module 31-reflector 29 pairs.

The reflector 29 is disposed such that a major axis direction X thereof is diagonal to one quadrant to be illuminated. In addition, the reflector 29 may be tilted at a predetermined angle α with respect to the horizontal surface HS, as shown in FIG. 7. The tilting angle α of the reflector 29 may be defined as an angle of the major axis direction X with respect to a horizontal direction, as shown in FIG. 9. The tilting angle α may be adjusted to increase the illuminance at a region of the horizontal surface near the lower column 21 or to increase the illuminance at a region of the horizontal surface far from the lower column 21. The illuminance at the region of the horizontal surface far from the lower column 21 can increase with increasing tilting angle α of the reflector 29. The tilting angle α may be in the range of greater than 0 degrees to less than 10 degrees, specifically in the range of 5 degrees to less than 10 degrees.

A reference point BP of the concave reflective surface is located directly above the light emitting module 31. A light beam reflected from the reference point BP after being emitted from a center of the light emitting module 31 is directed to a center of an illumination target area on the horizontal surface. An uppermost point of the concave reflective surface of the reflector 29 in a tilted state is referred to as a highest point HP. A light beam reflected from the highest point HP after being emitted from the center of the light emitting module 31 is delivered to a portion of the illumination target area on the horizontal surface, which is closer to the light emitting module 31 than the center of the illumination target area. In addition, a light beam L1 emitted from the light emitting module 31 towards a point near one end of a major diameter of the reflector is reflected towards a portion of the illumination target area far from the lower column 21 or the light emitting module 31, whereas a light beam L2 emitted from the light emitting module 31 towards a point near the other end of the major diameter is reflected toward a portion of the illumination target area near the lower column 21. As such, a light beam incident on a point on the concave reflective surface of the reflector 29, which is relatively close to the lower column 21 or the light emitting module 31, for example, the light beam L1, is reflected towards a portion of the illumination target area on the horizontal surface, which is relatively far from the lower column 21, whereas a light beam incident on a point on the concave reflective surface of the reflector 29, which is relatively far from the light emitting module 31, for example, the light beam L2, is reflected towards a portion of the illumination target area on the horizontal surface, which is relatively close to the lower column 21. Further, a light beam emitted from the light emitting module 31 towards the left side of the concave reflective surface with respect to the major axis direction X of the reflector 29 is reflected towards the right side of the illumination target area on the horizontal surface, whereas a light beam emitted from the light emitting module 31 towards the right side of the concave reflective surface is reflected towards the left side of the illumination target area on the horizontal surface. That is, light beams reflected from the concave reflective surface cross over one another.

Next, the structure of the concave reflective surface of the reflector 29 and an illumination area on the horizontal surface produced thereby will be described in more detail with reference to FIG. 9 and FIG. 10.

Referring to FIG. 9, the location of the light emitting module 31 is illustrated along with the reflector 29. The light emitting module 31 is disposed to be slightly offset to the left from the center of the reflector 29 in the major axis direction X. A point of the concave reflective surface located directly above the light emitting module 31 corresponds to the reference point BP. A point at which the major diameter Dx and minor diameter Dy of the reflector 29 intersect each other may be defined as a center of the reflector.

When the reflector 29 is disposed above the light emitting module 31 to be tilted at a predetermined angle α, the highest point HP of the concave reflective surface may be offset from the reference point BP towards the opposite end of the major diameter. That is, the reference point BP and the highest point HP may be separated from each other. In one embodiment, the highest point HP may be located directly above a point located between the center of the major diameter Dx and a point on the major diameter Dx, which is located directly above the light emitting module 31.

In FIG. 9, the points marked inside the reflector 29 are provided to illustrate an inner reflective surface of the concave reflective surface. For example, points closest to an edge of the reflector 29 correspond to an outermost rectangle among the rectangles shown in FIG. 10. That is, light beams reflected from the outermost points K1 of the concave reflective surface after being emitted from the light emitting module 31 are delivered to points on the outermost rectangle IK1 (see FIG. 10) on the horizontal surface. In addition, light beams reflected from the second outermost points K1 of the concave reflective surface after being emitted from the light emitting module 31 are delivered to points on the second outermost rectangle IK2 (see FIG. 10) on the horizontal surface. Further, light beams reflected from the third outermost points K1 of the concave reflective surface after being emitted from the light emitting module 31 are delivered to points on the third outermost rectangle IK3 (see FIG. 10) on the horizontal surface. As such, a light beam reflected from a point closer to the reference point BP of the concave reflective surface is delivered to a point closer to the center of a rectangular area on the horizontal surface. In particular, a light beam reflected from the reference point BP of the concave reflective surface is delivered to a center of the innermost rectangle among the rectangles shown in FIG. 10.

A light beam reflected from the one end of the major diameter, that is, a point P1 close to the light emitting module 31, after being emitted from the light emitting module 31 is delivered to a point IP1 on the horizontal surface far from the light emitting module 31. In addition, light beams reflected from points P2, P3, P4 (see FIG. 9) of the concave reflective surface are delivered to points IP2, IP3, IP4 on the horizontal surface, respectively. That is, light beams reflected from points of the concave reflective surface far from the light emitting module 31 are delivered to points on the horizontal surface close to the light emitting module 31, light beams reflected from the left side of the concave reflective surface with respect to the major diameter are delivered to the right side of the illumination target area on the horizontal surface, and light beams reflected from the right side of the concave reflective surface with respect to the major diameter are delivered to the left side of the illuminated target area on the horizontal surface. That is, light beams reflected from the reflector 29 may cross over one another. Accordingly, it is possible to reduce the size of the reflector 29 and to reduce the amount of light blocked by the support 23 after being reflected from the support 23. Since blockage of light caused by the support 23 can be minimized, it is possible to dispose the reflectors 29 closer to the center of the sterilization device 100, thereby reducing the overall size of the sterilization device 100.

The rectangular area of FIG. 10 is illuminated with light beams reflected from the inner reflective surface of the concave reflective surface of the reflector 29, which is surrounded by the outermost points K1. In addition, a region between the edge of the reflector 29 and the outermost points K1 may also reflect light beams. These light beams may be delivered to, for example, a region outside the rectangular area of FIG. 10.

In FIG. 9, the concave reflective surface is illustrated as being designed to illuminate a square area. However, it should be understood that the present disclosure is not limited thereto and the concave reflective surface may be designed to illuminate a rectangular area longer in one direction, or may be designed to illuminate an area having other geometric shapes.

FIG. 8 shows an illumination area on the horizontal surface HS produced by one light emitting module 31-reflector 29 pair. From FIG. 8, it can be seen that the illuminance level at a region close to the lower column 21 is generally higher than that at a region far from the lower column 21. However, the illumination arear has a generally rectangular shape. In addition, a point at which two diagonal lines in the generally rectangular area intersect each other will correspond to a point of incidence of light reflected from the reference point BP of the reflector 29.

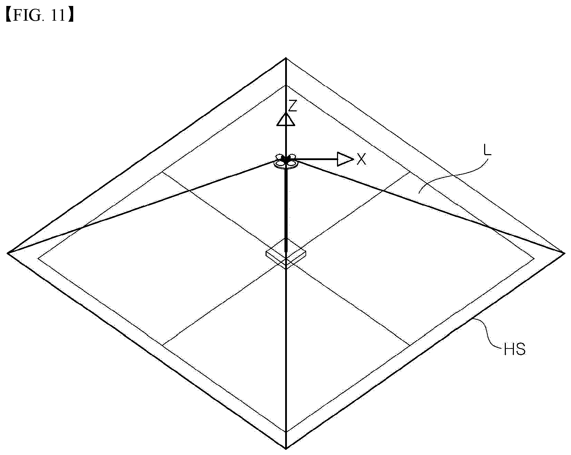

FIG. 11 is a schematic perspective view illustrating an illumination area produced by the UV sterilization device 100 according to the embodiment.

Referring to FIG. 11, four light emitting module 31-reflector 29 pairs may illuminate a horizontal surface HS having a generally rectangular shape. A space illuminated with light L reflected from the reflectors 29 has a pyramidal shape and the light L can sterilize pathogenic microorganisms in the space.

In particular, the sterilization device 100 according to the present disclosure performs sterilization when there is no human or animal in an indoor space. Accordingly, most pathogenic microorganisms floating in the indoor space will sink to a floor of the indoor space. Thus, the sterilization device 100 is suitable for sterilizing pathogenic microorganisms on the horizontal surface HS, that is, the floor.

The sterilization device 100 may be disposed at the center of the floor HS to uniformly illuminate the entire floor HS.

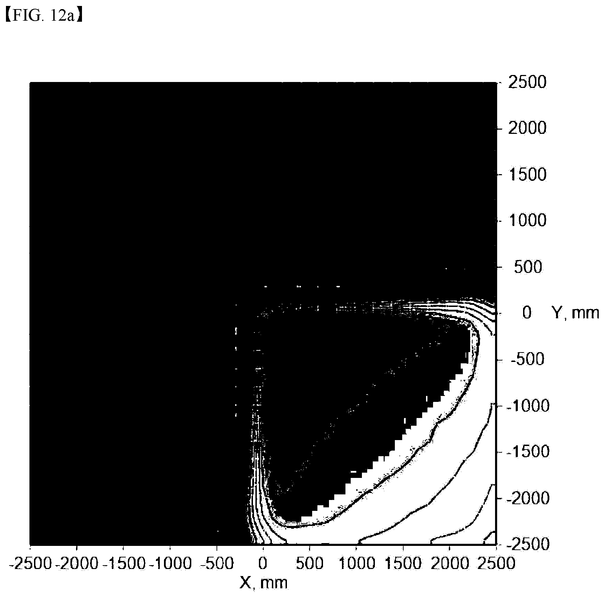

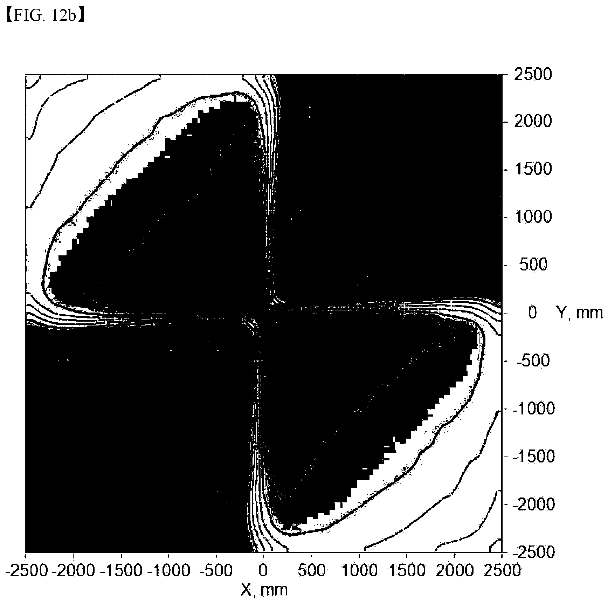

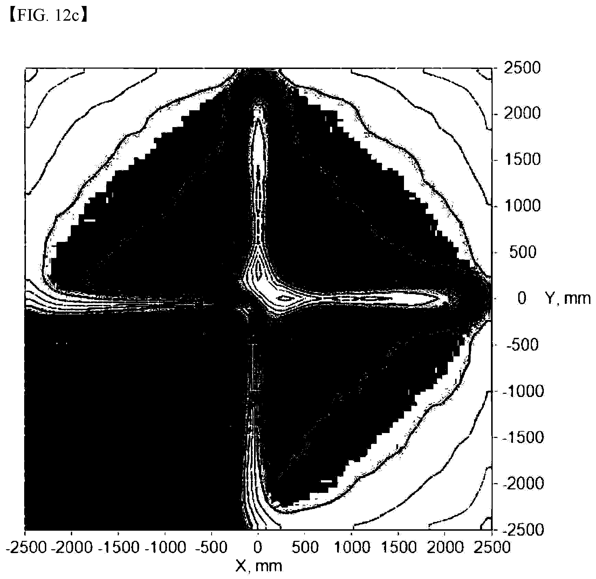

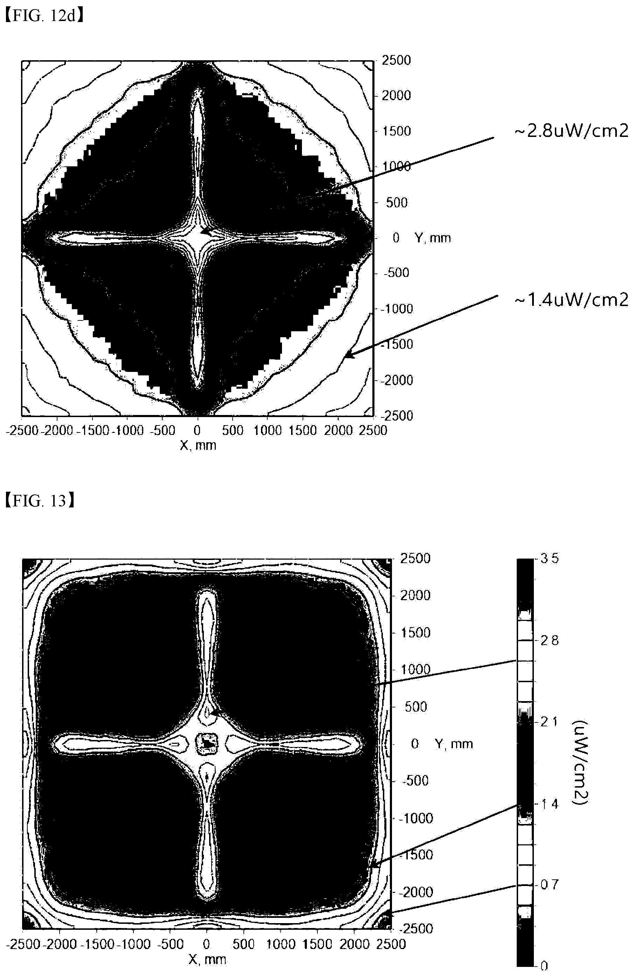

FIG. 12A, FIG. 12B, FIG. 12C, and FIG. 12D are respective simulation graphs of illuminance distributions on the floor produced by one, two, three, and four reflectors. Here, the major diameter of the reflector was 106.78 mm, the minor diameter of the reflector was 90.8 mm, the highest height of the concave reflective surface was 28.9 mm, and the tilting angle α of the reflector was about 5.7 degrees.

Referring to FIG. 12A, FIG. 12B, FIG. 12C, and FIG. 12D, it can be seen that all four quadrants of the floor can be illuminated by four light emitting module 31-reflector 29 pairs. In addition, as can be seen from the drawings, adjacent illumination areas may partially overlap one another other and thus the illuminance at the overlapping region may be relatively high.

FIG. 12D shows a distribution of illuminance on the floor with all the four quadrants thereof illuminated. It can be seen that illuminance level was highest (about 2.8 µW/cm²) at a central region of the floor and was lowest (less than 1.4 µW/cm²) at corners of the floor. As described above, light reflected from the reference point BP of one reflector 29 is delivered to the center of a corresponding one of the quadrants. The graph of FIG. 12D shows that the center of each quadrant has an illuminance of greater than 1.4 µW/cm². Accordingly, it can be seen that the maximum illuminance on the floor HS produced by the light emitting modules, that is, the illuminance at the central region of the floor, is less than twice as high as the illuminance at the center of each quadrant.

The distribution of illuminance according to this embodiment is more uniform than the distribution of illuminance of FIG. 1, produced by four light emitting modules disposed above the center of the floor as in this embodiment.

FIG. 13 is another simulation graph of an illuminance distribution on the floor produced by four reflectors.

In this embodiment, simulation was performed under the same conditions as in the embodiment of FIG. 12D, except that the tilting angle of the reflector was further increased by about 1.5 degrees to be set to about 7.2 degrees.

Referring to FIG. 13, it can be seen that increase in tilting angle α of the reflector 29 caused reduction in illuminance at the central region of the floor decreased and increase in illuminance at the corners of the floor, as compared with the simulation graph of FIG. 12D.

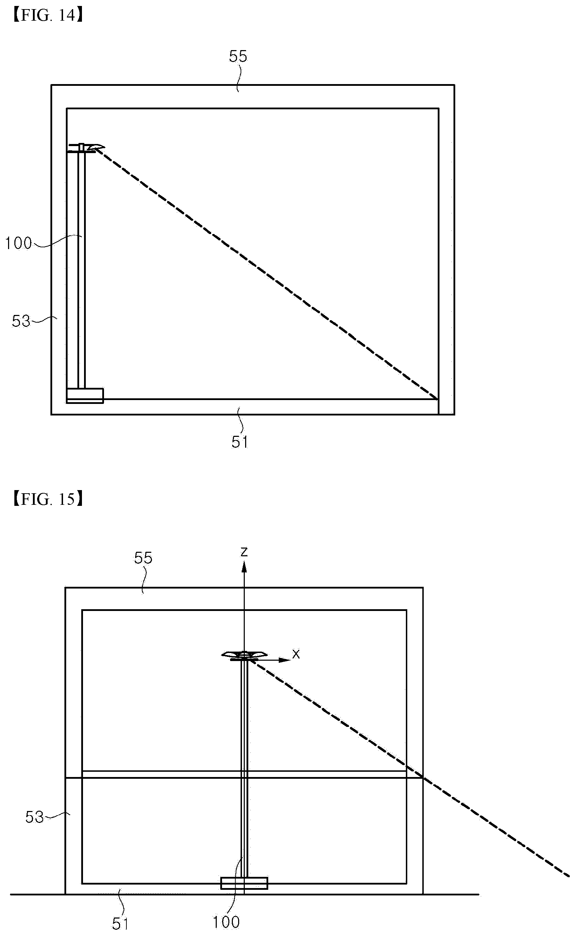

FIG. 14 is a schematic sectional view illustrating an exemplary application of the sterilization device 100 according to the embodiment.

The sterilization device 100 may be disposed near a wall 53 or at a corner where two walls meet to sterilize an indoor space and a floor 51. For example, when the sterilization device 100 is disposed near a center of the wall 53, the sterilization device 100 can sterilize the entire floor 51 using two light emitting module 31-reflector 29 pairs. When the sterilization device 100 is disposed at the corner, the sterilization device 100 can sterilize the floor 51 using one light emitting module 31-reflector 29 pair.

FIG. 15 is a schematic sectional illustrating another exemplary application of the sterilization device 100 according to the embodiment.

Referring to FIG. 15, the sterilization device 100 may be designed to sterilize a horizontal surface having a larger area than a floor 51 in a room so as to also sterilize a wall 53 in the room.

Light reflected from the concave reflective surface of the reflector 29 after being emitted from the light emitting module 31 generally propagates at an angle of up to about 55 degrees with respect to the lower column 21. Accordingly, it is possible to sterilize the wall 53 by placing the sterilization device 100 such that the wall 53 can be illuminated with light reflected from the reflector 29 at this angle.

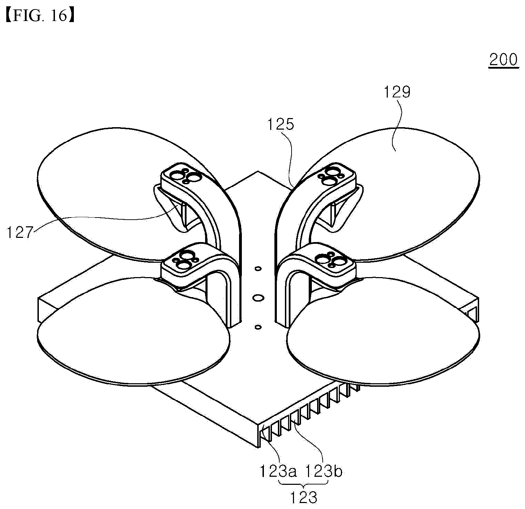

FIG. 16 is a schematic perspective view of a sterilization device 200 according to another embodiment of the present disclosure.

Referring to FIG. 16, the sterilization device 200 according to this embodiment is substantially the same as the sterilization device 100 described with reference to FIG. 1 to FIG 5, except that the sterilization device 200 employs a different support 123 and a different connection structure between the support 123 and reflectors 129 than the sterilization device 100.

The support 23 shown in FIG. 1 to FIG. 5 has a plate-like shape, whereas the support 123 in this embodiment includes multiple heat dissipation fins 123b along with a plate 123a. The heat dissipation fins 123b can facilitate cooling of the light emitting modules 31.

Each of the reflectors 129 may be coupled to the support 123 using a connector 125 and a bracket 127 instead of using the upper column 25 and the connecting beam 27 in the above embodiment.

The bracket 127 is disposed on an upper surface of the reflector 129 and the connector 125 is coupled to the support 123. The connector 125 and the bracket 127 may be fastened to each other by a screw.

Although various embodiments of the light emitting module have been described herein, it should be understood that the present disclosure is not limited thereto and may be embodied in different ways. In addition, the light emitting module set forth above may be used as a light source providing uniform planar illumination in display devices, such as liquid crystal displays, or lighting devices.

## Claims

1. A sterilization device comprising:
a light emitting module (31); and
a reflector (29) reflecting light emitted from the light emitting module,
wherein the light emitting module comprises a light emitting diode emitting sterilizing light,
wherein the reflector comprises a concave reflective surface reflecting the light emitted from the light emitting module towards a horizontal surface (HS) below the light emitting module, and
wherein a major axis direction (X) refers to a direction of a maximum diameter (Dx) of the reflector, and wherein this diameter (Dx) is referred to as a major diameter,
wherein the concave reflective surface has a smaller radius of curvature near one end (P1) of the major diameter (Dx) thereof than near the other end of the major diameter thereof,
wherein the concave reflective surface is disposed above the light emitting module such that the major diameter thereof extends over the light emitting module, and the light emitting module is disposed closer to the one end of the major diameter of the concave reflective surface than to the other end of the major diameter of the concave reflective surface,
wherein a major axis direction of the concave reflective surface is tilted with respect to the horizontal surface such that the one end of the major diameter of the concave reflective surface is closer to the horizontal surface than the opposite end of the major diameter of the concave reflective surface,
**characterized in that**
an uppermost point (HP) of the concave reflective surface tilted with respect to the horizontal surface is located between a center of the major diameter of the concave reflective surface and a reference point (BP) of the concave reflective surface located directly above the light emitting module.

2. The sterilization device according to claim 1, wherein the concave reflective surface has a minor diameter (Dy) orthogonal to the major diameter, the minor diameter being parallel to the horizontal surface.

3. The sterilization device according to claim 1, wherein the concave reflective surface comprises an inner reflective surface reflecting the light emitted from the light emitting module towards a rectangular area on the horizontal surface.

4. The sterilization device according to claim 3, wherein the rectangular area is square.

5. The sterilization device according to claim 3, wherein a light beam reflected from the reference point of the concave reflective surface located directly above the light emitting module is directed to a center of the rectangular area.

6. The sterilization device according to claim 5, wherein a light beam reflected from the uppermost point of the concave reflective surface is delivered to a region between the center of the rectangular area and a vertical projection point of the light emitting module on the rectangular area.

7. The sterilization device according to claim 6, wherein:
a light beam reflected from an edge of the inner reflective surface closest to the one end (P1) of the major diameter of the concave reflective surface among edges of the inner reflective surface is delivered to a vertex of the rectangular area farthest from the light emitting module among vertices of the rectangular area; and
a light beam reflected from an edge of the inner reflective surface closest to the other end of the major diameter of the concave reflective surface among the edges of the internal reflection surface is delivered to a vertex of the rectangular area closest to the light emitting module among the vertices of the rectangular area.

8. The sterilization device according to claim 1, further comprising:
a support (23) supporting the light emitting module.

9. The sterilization device according to claim 1, wherein a maximum illuminance on the horizontal surface illuminated with the sterilizing light is less than twice as high as an illuminance at a point on the horizontal surface illuminated with a light beam reflected from a reference point of the concave reflective surface located directly above the light emitting module.

10. The sterilization device according to claim 1, comprising:
four light emitting modules; and
four reflectors each reflecting light emitted from a corresponding one of the four light emitting modules.

11. The sterilization device according to claim 10, wherein the four light emitting modules and the four reflectors are arranged to illuminate four quadrants of the horizontal surface about the sterilization device, respectively.

12. The sterilization device according to claim 14, wherein an illuminance at a center of the horizontal surface illuminated with the sterilizing light is less than twice as high as an illuminance at a point on the horizontal surface illuminated with a light beam reflected from a reference point of the concave reflective surface located directly above each of the light emitting modules.

## Patentansprüche

1. Sterilisationsvorrichtung, umfassend:
ein lichtemittierendes Modul (31); und
einen Reflektor (29), der das vom lichtemittierenden Modul emittierte Licht reflektiert,
wobei das lichtemittierendes Modul eine lichtemittierende Diode umfasst, die Sterilisationslicht emittiert,
wobei der Reflektor eine konkave reflektierende Oberfläche umfasst, die das vom lichtemittierenden Modul emittierte Licht in Richtung einer horizontalen Fläche (HS) unterhalb des lichtemittierenden Moduls reflektiert, und
wobei sich eine Hauptachsenrichtung (X) auf eine Richtung eines maximalen Durchmessers (Dx) des Reflektors bezieht und wobei dieser Durchmesser (Dx) als Hauptdurchmesser bezeichnet wird,
wobei die konkave reflektierende Oberfläche in der Nähe eines Endes (P1) ihres Hauptdurchmessers (Dx) einen kleineren Krümmungsradius aufweist als in der Nähe des anderen Endes ihres Hauptdurchmessers,
wobei die konkave reflektierende Oberfläche vor dem lichtemittierenden Modul so angeordnet ist, dass sich ihr Hauptdurchmesser über das lichtemittierende Modul erstreckt, und das lichtemittierende Modul näher an dem einen Ende des Hauptdurchmessers der konkaven reflektierenden Oberfläche angeordnet ist als an dem anderen Ende des Hauptdurchmessers der konkaven reflektierenden Oberfläche,
wobei eine Hauptachsenrichtung der konkaven reflektierenden Oberfläche in Bezug auf die horizontale Oberfläche so geneigt ist, dass das eine Ende des Hauptdurchmessers der konkaven reflektierenden Oberfläche näher an der horizontalen Oberfläche liegt als das gegenüberliegende Ende des Hauptdurchmessers der konkaven reflektierenden Oberfläche,
**dadurch gekennzeichnet, dass**
ein höchster Punkt (HP) der gegenüber der horizontalen Fläche geneigten konkaven reflektierenden Fläche zwischen einem Mittelpunkt des Hauptdurchmessers der konkaven reflektierenden Fläche und einem Referenzpunkt (BP) der konkaven reflektierenden Fläche liegt, der sich direkt vor dem lichtemittierenden Modul befindet.

2. Sterilisationsvorrichtung nach Anspruch 1, wobei die konkave reflektierende Oberfläche einen zum Hauptdurchmesser orthogonalen Nebendurchmesser (Dy) aufweist, wobei der Nebendurchmesser parallel zur horizontalen Fläche verläuft.

3. Sterilisationsvorrichtung nach Anspruch 1, wobei die konkave reflektierende Fläche eine innere reflektierende Fläche umfasst, die das vom lichtemittierenden Modul emittierte Licht in Richtung eines rechteckigen Bereichs auf der horizontalen Fläche reflektiert.

4. Sterilisationsvorrichtung nach Anspruch 3, wobei der rechteckige Bereich quadratisch ist.

5. Sterilisationsvorrichtung nach Anspruch 3, wobei ein Lichtstrahl, der vom Bezugspunkt der direkt vor dem lichtemittierenden Modul angeordneten konkaven reflektierenden Oberfläche reflektiert wird, auf einen Mittelpunkt des rechteckigen Bereichs gerichtet ist.

6. Sterilisationsvorrichtung nach Anspruch 5, wobei ein vom obersten Punkt der konkaven reflektierenden Oberfläche reflektierter Lichtstrahl auf einen Bereich zwischen dem Mittelpunkt des rechteckigen Bereichs und einem vertikalen Projektionspunkt des lichtemittierenden Moduls auf dem rechteckigen Bereich gerichtet wird.

7. Sterilisationsvorrichtung nach Anspruch 6, wobei:
ein Lichtstrahl, der von einer Kante der inneren reflektierenden Oberfläche reflektiert wird, die unter den Kanten der inneren reflektierenden Oberfläche dem einen Ende (P1) des Hauptdurchmessers der konkaven reflektierenden Oberfläche am nächsten liegt, auf einen Scheitelpunkt des rechteckigen Bereichs geleitet wird, der unter den Scheitelpunkten des rechteckigen Bereichs am weitesten v m vom lichtemittierenden Modul entfernt ist; und
ein Lichtstrahl, der von einer Kante der inneren reflektierenden Oberfläche reflektiert wird, die unter den Kanten der inneren reflektierenden Oberfläche dem anderen Ende des Hauptdurchmessers der konkaven reflektierenden Oberfläche am nächsten liegt, zu einem Eckpunkt des rechteckigen Bereichs geleitet wird, der unter den Eckpunkten des rechteckigen Bereichs dem lichtemittierenden Modul am nächsten liegt.

8. Sterilisationsvorrichtung nach Anspruch 1, ferner umfassend:
eine Halterung (23), die das lichtemittierende Modul trägt.

9. Sterilisationsvorrichtung nach Anspruch 1, wobei eine maximale Beleuchtungsstärke auf der mit dem Sterilisationslicht beleuchteten horizontalen Fläche weniger als doppelt so hoch ist wie eine Beleuchtungsstärke an einem Punkt auf der horizontalen Fläche, der mit einem Lichtstrahl beleuchtet wird, der von einem Referenzpunkt der konkaven reflektierenden Oberfläche reflektiert wird, der sich direkt über dem lichtemittierenden Modul befindet.

10. Sterilisationsvorrichtung nach Anspruch 1, umfassend:
vier lichtemittierende Module; und
vier Reflektoren, von denen jeder das von einem der vier lichtemittierenden Module ausgestrahlte Licht reflektiert.

11. Sterilisationsvorrichtung nach Anspruch 10, wobei die vier lichtemittierenden Module und die vier Reflektoren so angeordnet sind, dass sie jeweils vier Quadranten der horizontalen Fläche um die Sterilisationsvorrichtung herum beleuchten.

12. Sterilisationsvorrichtung nach Anspruch 11, wobei eine Beleuchtungsstärke an einem Mittelpunkt der horizontalen Fläche, der mit dem Sterilisationslicht beleuchtet wird, weniger als doppelt so hoch ist wie eine Beleuchtungsstärke an einem Punkt auf der horizontalen Fläche, der mit einem Lichtstrahl beleuchtet wird, der von einem Referenzpunkt der konkaven reflektierenden Oberfläche reflektiert wird, der sich direkt über jedem der lichtemittierenden Module befindet.

## Revendications

1. Dispositif de stérilisation comprenant :
un module d'émission de lumière (31) ; et
un réflecteur (29) réfléchissant la lumière émise par le module d'émission de lumière,
dans lequel le module d'émission de lumière comprend une diode électroluminescente émettant une lumière stérilisante,
dans lequel le réflecteur comprend une surface réfléchissante concave réfléchissant la lumière émise par le module d'émission de lumière vers une surface horizontale (HS) située en dessous du module d'émission de lumière, et
dans lequel une direction d'axe principal (X) désigne une direction d'un diamètre maximal (Dx) du réflecteur, et dans lequel ce diamètre (Dx) est appelé diamètre principal,
dans lequel la surface réfléchissante concave présente un rayon de courbure plus petit près d'une extrémité (P1) de son diamètre principal (Dx) que près de l'autre extrémité de son diamètre principal,
dans lequel la surface réfléchissante concave est disposée au-dessus du module d'émission de lumière de telle sorte que son diamètre principal s'étende au-dessus du module d'émission de lumière, et le module d'émission de lumière est disposé plus près de l'une des extrémités du diamètre principal de la surface réfléchissante concave que de l'autre extrémité du diamètre principal de la surface réfléchissante concave,
dans lequel une direction de l'axe principal de la surface réfléchissante concave est inclinée par rapport à la surface horizontale de telle sorte que ladite une extrémité du diamètre principal de la surface réfléchissante concave soit plus proche de la surface horizontale que l'extrémité opposée du diamètre principal de la surface réfléchissante concave,
**caractérisé en ce que**
un point le plus haut (HP) de la surface réfléchissante concave inclinée par rapport à la surface horizontale est situé entre un centre du grand diamètre de la surface réfléchissante concave et un point de référence (BP) de la surface réfléchissante concave situé directement au-dessus du module d'émission de lumière.

2. Dispositif de stérilisation selon la revendication 1, dans lequel la surface réfléchissante concave présente un petit diamètre (Dy) orthogonal au grand diamètre, le petit diamètre étant parallèle à la surface horizontale.

3. Dispositif de stérilisation selon la revendication 1, dans lequel la surface réfléchissante concave comprend une surface réfléchissante interne réfléchissant la lumière émise par le module d'émission de lumière vers une zone rectangulaire sur la surface horizontale.

4. Dispositif de stérilisation selon la revendication 3, dans lequel la surface rectangulaire est carrée.

5. Dispositif de stérilisation selon la revendication 3, dans lequel un faisceau lumineux réfléchi par le point de référence de la surface réfléchissante concave située directement au-dessus du module émetteur de lumière est dirigé vers le centre de la zone rectangulaire.

6. Dispositif de stérilisation selon la revendication 5, dans lequel un faisceau lumineux réfléchi par le point le plus haut de la surface réfléchissante concave est dirigé vers une région située entre le centre de la zone rectangulaire et un point de projection verticale du module d'émission de lumière sur la zone rectangulaire.

7. Dispositif de stérilisation selon la revendication 6, dans lequel :
un faisceau lumineux réfléchi par un bord de la surface réfléchissante interne le plus proche de l'une des extrémités (P1) du grand axe de la surface réfléchissante concave parmi les bords de la surface réfléchissante interne est dirigé vers un sommet de la zone rectangulaire le plus éloigné du module d'émission de lumière parmi les sommets de la zone rectangulaire ; et
un faisceau lumineux réfléchi par un bord de la surface réfléchissante interne le plus proche de l'autre extrémité du grand diamètre de la surface réfléchissante concave parmi les bords de la surface réfléchissante interne est dirigé vers un sommet de la zone rectangulaire le plus proche du module d'émission de lumière parmi les sommets de la zone rectangulaire.

8. Dispositif de stérilisation selon la revendication 1, comprenant en outre :
un support (23) supportant le module d'émission de lumière.

9. Dispositif de stérilisation selon la revendication 1, dans lequel l'éclairement maximal sur la surface horizontale éclairée par la lumière de stérilisation est inférieur à deux fois l'éclairement en un point de la surface horizontale éclairé par un faisceau lumineux réfléchi par un point de référence de la surface réfléchissante concave situé directement au-dessus du module d'émission de lumière.

10. Dispositif de stérilisation selon la revendication 1, comprenant :
quatre modules d'émission de lumière ; et
quatre réflecteurs, chacun réfléchissant la lumière émise par l'un des quatre modules d'émission de lumière correspondants.

11. Dispositif de stérilisation selon la revendication 10, dans lequel les quatre modules d'émission de lumière et les quatre réflecteurs sont agencés de manière à assurer l'illumination respectivement de quatre quadrants de la surface horizontale autour du dispositif de stérilisation.

12. Dispositif de stérilisation selon la revendication 11, dans lequel l'éclairement au centre de la surface horizontale éclairée par la lumière de stérilisation est inférieur à deux fois l'éclairement en un point de la surface horizontale éclairé par un faisceau lumineux réfléchi par un point de référence de la surface réfléchissante concave situé directement au-dessus de chacun des modules d'émission de lumière.
